## Europäisches Patentamt
## European Patent Office
## Office européen des brevets

(11) Veröffentlichungsnummer: **0 193 174**
**B1**

(12) # EUROPÄISCHE PATENTSCHRIFT

(45) Veröffentlichungstag der Patentschrift:
02.08.89

(51) Int. Cl.⁴: **A 61 M 16/01**

(21) Anmeldenummer: **86102459.4**

(22) Anmeldetag: **25.02.86**

(54) Anordnung zum Abpumpen von Ausatmungsgas eines Patienten.

(30) Priorität: **26.02.85 DE 3506674**

(43) Veröffentlichungstag der Anmeldung:
**03.09.86 Patentblatt 86/36**

(45) Bekanntmachung des Hinweises auf die Patenterteilung:
**02.08.89 Patentblatt 89/31**

(84) Benannte Vertragsstaaten:
**CH DE FR GB IT LI SE**

(56) Entgegenhaltungen:
**DE-A-2 942 623**
**FR-A-2 395 036**
**GB-A-1 446 432**
**GB-A-2 040 690**

(73) Patentinhaber: **Siemens- Elema AB, Röntgenvägen 2, S-171 95 Solna 1 (SE)**
(84) Benannte Vertragsstaaten: **SE**

(73) Patentinhaber: **SIEMENS AKTIENGESELLSCHAFT, Postfach 22 16 34, D-8000 München 22 (DE)**
(84) Benannte Vertragsstaaten: **CH DE FR GB IT LI**

(72) Erfinder: **Westerberg, Hans, Lummergangen 16, S-135 35 Tyresoe (SE)**

EP 0 193 174 B1

## Beschreibung

Die Erfindung betrifft eine Anordnung zum Abpumpen von Ausatmungsgas eines Patienten über ein Beatmunsgerät mit einer Gasleitung, die die Gasaustrittsöffnung des Beatmungsgerätes mit einem Auffangbehälter für das abgepumpte Gas verbindet und wobei die Gasleitung über eine Öffnung mit der Atmosphäre verbindbar ist.

Eine Anordnung dieser Art ist durch den Prospekt der Fa. Vital Signs Inc. von 1982 bekannt. Die Anordnung ist an eine Saugquelle, z. B. einen Ejektor geschaltet, der das Gas mit einer kontinuierlichen, bestimmten Geschwindigkeit aus der Gasleitung herausbefördert. An der Gasleitung ist ferner eine Gummiblase angeschlossen, die dazu dient, kurzzeitigen Überdruck in der Gasleitung auszugleichen. Wenn der Patient kräftig ausatmet, entsteht in der Gasleitung ein Überdruck, der sich dadurch abbaut, dass die Gummiblase mit Gas gefüllt wird. Die Saugquelle ist immer so eingestellt, dass sie bei normaler Ausatmung des Patienten einen schwachen Unterdruck in der Gasleitung erzeugt. Daher wird das Gas in der Gummiblase bei dem nächsten Atemzyklus aus dieser herausgetrieben. Um bei normaler oder ausbleibender Ausatmung einen hohen Unterdruck in der Gasleitung zu vermeiden, ist sie über eine Öffnung mit der Atmosphäre verbunden.

Ein Beatmungsgerät der eingangs genannten Art weist eine Alarmanordnung auf, die bei einem Fehler im System ausgelöst wird. So ist ein Flussmessgerät, das im Bereich der Gasaustrittsöffnung des Beatmungsgerätes angebracht ist, mit der Alarmanordnung verbunden. Beim Unterschreiten eines bestimmten Gasflusses wird der Alarm ausgelöst. Wenn sich nun aber der Ausatmungsschlauch vom Patienten zum Beatmungsgerät löst, schaltet die Alarmanordnung nicht ein, da die Saugquelle statt Ausatmungsgas über das Flussmessgerät Atmosphärenluft absaugt. Dies stellt, wenn es nicht rechtzeitig entdeckt wird, eine Gefahr für den Patienten dar.

Der Erfindung liegt die Aufgabe zugrunde, eine Anordnung der eingangs genannten Art zu schaffen, durch die auch bei einem Leck in der Verbindung zwischen Patienten und Flussmessgerät die Alarmfunktion des Beatmungsgerätes ausgelöst wird.

Diese Aufgabe ist erfindungsgemäss dadurch gelöst, dass in der Gasleitung zwischen der Gasaustrittsöffnung und der Öffnung zum Verbinden der Gasleitung mit der Atmosphäre ein Ventil angeordnet ist, das beim Unterschreiten des Druckes in der Gasleitung zwischen Ventil und Gasaustrittsöffnung unter einen vorgebbaren Wert die Verbindung von der Gasaustrittsöffnung zum Auffangbehälter unterbricht. Wegen der Unterbrechung der Gaszufuhr vom Beatmungsgerät zum Auffangbehälter beim Ausbleiben des Ausatmungsgases vom Patienten zeigt das Flussmessgerät gleich einen Wert an, der den Alarm auslöst. Die Saugquelle saugt nunmehr Gas über die Öffnung der Gasleitung zur Atmosphäre an.

In einer vorteilhaften Weiterbildung der Erfindung wird vorgeschlagen, dass zwischen Gasaustrittsöffnung und Ventil ein aufblasbares Reservoir an die Gasleitung angeschlossen ist, über das das Ventil gesteuert wird. Das Reservoir kann z. B. ein Balg sein, der so mit dem Ventil verbunden ist, dass dieses bei einem gefüllten Balg offen und bei einem leeren gesperrt ist.

In einer weiteren Ausgestaltung der Erfindung wird vorgeschlagen, dass das Gasreservoir eine Gummiblase ist, an deren Öffnung das Ventil mit einem Gehäuse und einem Ventilkörper angebracht ist. Als Betätigungsteil für das Ventil ist eine Stange vorgesehen, die im Inneren durch die gesamte Länge der Gummiblase ragt und am geschlossenen Ende an dieser befestigt ist. Dadurch ist mit einfachen konstruktiven Mitteln erreicht, dass das Ventil, abhängig von der Gasmenge in der Gummiblase und damit vom Gasdruck zwischen Ventil und Gasaustrittsöffnung, geöffnet oder geschlossen wird. Durch die wenigen Teile des Ventils ist dieses im Vergleich zu pneumatisch gesteuerten, die häufig durch Verunreinigungen verstopft werden, auch leicht zu reinigen.

In einer weiteren Ausbildung der Anordnung wird vorgeschlagen, dass der Ventilkörper kegelförmig ausgebildet ist. Durch die Form ist ein genaues Einrasten des Ventilkörpers im Ventilsitz und damit eine gute Abdichtung gegeben.

Eine technisch einfache Lösung ergibt sich dadurch, dass die Ventilstange elastisch biegbar ist. Die Ventilstange kann dadurch einer unsachgemässen seitlichen Verschiebung der Gummiblase folgen.

Die Erfindung ist nachfolgend anhand eines in den Zeichnungen dargestellten Ausführungsbeispiels näher erläutert. Es zeigen:

Fig. 1 eine schematische Darstellung einer Anordnung nach der Erfindung in Verbindung mit einem bekannten Beatmungsgerät und

Fig. 2 einen Querschnitt durch die Anordnung nach Fig. 1.

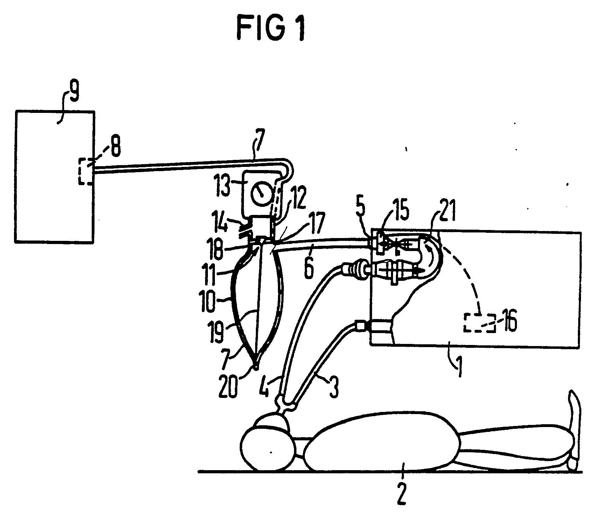

In der Figur 1 wird ein Beatmungsgerät 1 gezeigt, an dem ein Patient 2 über einen Einatmungsschlauch 3 und einen Ausatmungsschlauch 4 angeschlossen ist. Das Beatmungsgerät 1 ist über seine Gasaustrittsöffnung 5 mit einer Anordnung zum Abpumpen von Ausatmungsgas des Patienten 2 verbunden. Diese Anordnung weist eine Gasleitung 6 auf, die über eine weitere Gasleitung 7 mit einer Saugquelle 8 zum kontinuierlichen Absaugen des Gases und einem Auffangbehälter 9 für das abgepumpte Gas verbunden ist. Zwischen den Gasleitungen 6 und 7 ist ein später näher beschriebenes Gasreservoir 10 mit einem Ventil 11 angebracht. Das Gasreservoir 10 ist an einem Gehäuse 12 mit einem Flussmessgerät 13 befestigt. Das Flussmessgerät 13 zeigt den Gasfluss im System hinter dem Ventil 11 an und dient u.a. zur Kontrolle, ob die Saugquelle 8 einwandfrei funktioniert. Das Gehäuse 12 weist ferner eine Öffnung 14 zur Atmosphäre auf. Das Beatmungsgerät 1 besitzt im Bereich der Austrittsöffnung 5 ein weiteres Flussmessgerät 15, das mit einer Alarmanordnung 16 verbunden ist.

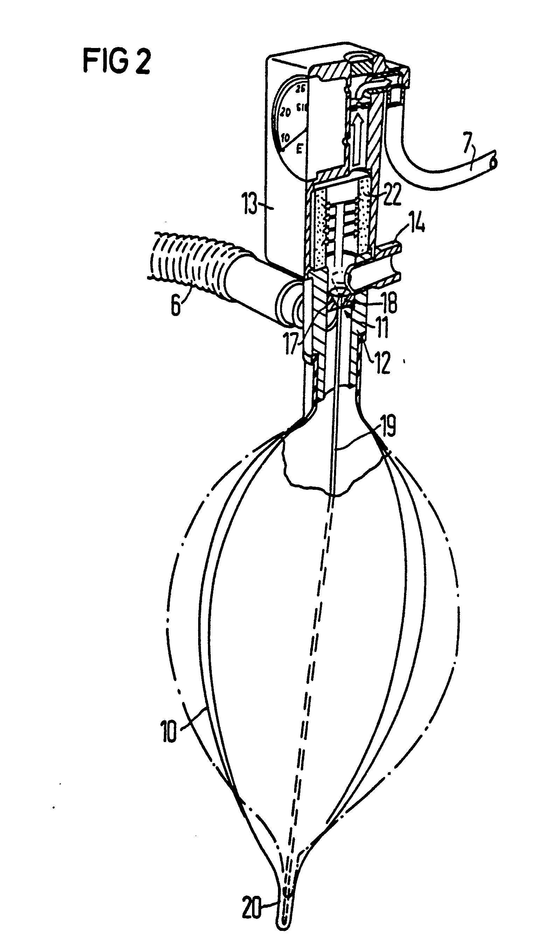

In der Figur 2 ist näher dargestellt, dass das Gasreservoir 10 aus einer Gummiblase besteht und am dem Gehäuse 12 befestigt ist. Das Ventil 11 - bestehend aus einem Ventilsitz 17 und einem kegelförmigen Ventilkörper 18 - weist eine elastisch biegbare Stange 19 auf, die als Betätigungsteil für das Ventil 11 dient. Die Ventilstange 19, die durch die gesamte Länge der Gummiblase 10 ragt, ist am geschlossenen Ende 20 an dieser befestigt.

Die Funktion der Gummiblase 10 mit dem Ventil 11 wird nun anhand der Figuren 1 und 2 näher beschrieben. Dem Patienten 2 wird vom Beatmungsgerät 1 über den Einatmungsschlauch 3 Einatmungsgas zugeführt. Das Ausatmungsgas wird über den Schlauch 4 und eine Verbindungsleitung 21 im Beatmungsgerät und weiterhin über die Gasleitung 6 zur Gummiblase 10 geleitet. Das Flussmessgerät 15 registriert dabei den Gasfluss durch die Verbindungsleitung 21. Die Gummiblase 10 wird durch das Gas ausgedehnt und damit kürzer, wodurch der Ventilkörper 18 - wie in Figur 2 strichpunktiert angedeutet - vom Ventilsitz 17 abhebt. Das Ausatmungsgas strömt zunächst durch ein im Gehäuse 12 vorhandenes Filter 22 und wird danach über die Gasleitung zum Auffangbehälter 9 geleitet. Das Filter 22 dient dazu, Schmutzpartikel von der Atmosphärenluft aufzufangen. Wenn die Gummiblase 10 vom Gas entleert ist, geht sie in ihre ursprüngliche Lage zurück, wobei der Ventilkörper 17 die Gaszufuhr sperrt.

Wenn ein Leck in der Verbindung zwischen Patienten 2 und Flussmessgerät 15 vorhanden ist, fällt der Überdruck in der Gummiblase 10 ab, wobei das Ventil 11 schliesst. Die Saugquelle 8 erhält nunmehr Atmosphärengas durch die Öffnung 14. Der Gasfluss in den Leitungen 6 bzw. 21 Sinkt dabei unter einen bestimmten Grenzwert, bei dem der Flussmesser 15 die Alarmanordnung 16 auslöst. Auf diese Art kann eine Bedienungsperson rechtzeitig den Fehler entdecken, ohne dass der Patient zu Schaden kommt.

**Patentansprüche**

1. Anordnung zum Abpumpen von Ausatmungsgas eines Patienten über ein Beatmungsgerät (1) mit einer Gasleitung (6), die die Gasaustrittsöffnung (5) des Beatmungsgerätes mit einem Auffangbehälter (9) für das abgepumpte Gas verbindet, wobei die Gasleitung über eine Öffnung (14) mit der Atmosphäre verbindbar ist, dadurch gekennzeichnet, dass in der Gasleitung (6) zwischen der Gasaustrittsöffnung (5) und der Öffnung (14) zum Verbinden der Gasleitung (6) mit der Atmosphäre ein Ventil (11) angeordnet ist, das beim Unterschreiten des Druckes in der Gasleitung (6) zwischen Ventil (11) und Gasaustrittsöffnung (5) unter einen vorgebbaren Wert die Verbindung von der Gasaustrittsöffnung (5) zum Auffangbehälter (9) unterbricht.

2. Anordnung nach Anspruch 1, dadurch gekennzeichnet, dass zwischen Gasaustrittsöffnung (5) und Ventil (11) ein aufblasbares Reservoir (10) an die Gasleitung (6) angeschlossen ist, über das das Ventil gesteuert wird.

3. Anordnung nach Anspruch 2, dadurch gekennzeichnet, dass das Gasreservoir (10) eine Gummiblase ist, an deren Öffnung das Ventil (11) mit einem Ventilsitz (17) und einem Ventilkörper (18) angebracht ist, und dass als Betätigungsteil für den Ventilkörper (18) eine Stange (19) vorgesehen ist, die im Inneren durch die gesamte Länge der Gummiblase (10) ragt und am geschlossenen Ende (20) an dieser befestigt ist.

4. Anordnung nach Anspruch 3, dadurch gekennzeichnet, dass der Ventilkörper (18) kegelförmig ausgebildet ist.

5. Anordnung nach Anspruch 3, dadurch gekennzeichnet, dass die Ventilstange (19) elastisch biegbar ist.

**Claims**

1. Evacuation arrangement for gases exhaled by a patient through a respirator (1) having a gas line (6) which connects the gas outlet orifice (5) of the respirator to a receiving tank (9) for the evacuated gas, the gas line being connectable through an orifice (14) to the atmosphere, characterized in that a valve (11) is arranged in the gas line (6) between the gas outlet orifice (5) and the orifice (14) for connecting the gas line (6) to the atmosphere, and interrupts the connection from the gas outlet orifice (5) to the receiving tank (9) when the pressure in the gas line (6) between valve (11) and gas outlet orifice (5) falls below a predeterminable value.

2. Arrangement according to Claim 1, characterized in that an inflatable reservoir (10), through which the valve is controlled, is connected to the gas line (6) between gas outlet orifice (5) and valve (11).

3. Arrangement according to Claim 2, characterized in that the gas reservoir (10) is a rubber bladder, to the orifice of which the valve (11) is attached with a valve seat (17) and a valve element (18), and that a rod (19) which is provided as actuating part for the valve element (18) projects through the entire length of the rubber bladder (10) on the inside and is fastened to the latter at the closed end (20).

4. Arrangement according to Claim 3, characterized in that the valve element (18) is of conical construction.

5. Arrangement according to Claim 3, characterized in that the valve rod (19) is resiliently flexible.

## Revendications

1. Dispositif d'évacuation du gaz expiré par un patient par un appareil respiratoire (1), comprenant un conduit pour le gaz (6), qui met l'orifice de sortie du gaz (5) de l'appareil respiratoire en communication avec la cuve de réception (9) du gaz évacué, le conduit pour le gaz pouvant communiquer avec l'atmosphère par un orifice (14), caractérisé en ce que dans le conduit pour le gaz (6) est monté, entre l'orifice de sortie du gaz (5) et l'orifice (14) mettant le conduit pour le gaz (6) en communication avec l'atmosphère, une soupape (11) qui interrompt la communication entre l'orifice de sortie du gaz et la cuve de réception (9) lorsque la pression dans le conduit pour le gaz (6) entre la soupape (11) et l'orifice de sortie du gaz (5) devient inférieure à une valeur prescrite.

2. Dispositif suivant la revendication 1, caractérisé en ce que, entre l'orifice de sortie du gaz (5) et la soupape (11) est raccordé au conduit pour le gaz (6) un réservoir (10) qui est gonflable et par lequel la soupape est commandée.

3. Dispositif suivant la revendication 2, caractérisé en ce que le réservoir à gaz (10) est une vessie en caoutchouc sur l'ouverture de laquelle est montée la soupape (11) ayant un siège de soupape (17) et un corps de soupape (18), et en ce que, comme pièce de manoeuvre du corps de soupape (18), il est prévu une tige (19) qui fait saillie à l'intérieur sur toute la longueur de la vessie en caoutchouc (10) et qui est fixée à celle-ci, à l'extrémité fermée (20).

4. Dispositif suivant la revendication 3, caractérisé en ce que le corps de soupape (18) est de forme conique.

5. Dispositif suivant la revendication 3, caractérisé en ce que la tige de soupape (19) peut se incurver élastiquement.

# FIG 1

FIG 2